Europäisches Patentamt

**(19)** European Patent Office

Office européen des brevets

**(11)** Publication number : **0 326 196 B1**

**(12)** ## EUROPEAN PATENT SPECIFICATION

**(45)** Date of publication of patent specification :
25.03.92 Bulletin 92/13

**(51)** Int. Cl.⁵ : **A61K 31/575,** A61K 47/00,
A61K 31/57

**(21)** Application number : **89200039.9**

**(22)** Date of filing : **09.01.89**

**(54)** Aqueous pharmaceutical preparation.

**(30)** Priority : **14.01.88 US 143888**

**(43)** Date of publication of application :
**02.08.89 Bulletin 89/31**

**(45)** Publication of the grant of the patent :
**25.03.92 Bulletin 92/13**

**(84)** Designated Contracting States :
**AT BE CH DE ES FR GB GR IT LI LU NL SE**

**(56)** References cited :
**EP-A- 0 149 197**
**FR-A- 2 484 252**
**US-A- 4 383 992**
**US-A- 4 686 214**

**(73)** Proprietor : **AKZO N.V.**
**Velperweg 76**
**NL-6824 BM Arnhem (NL)**

**(72)** Inventor : **Brinks, Gerrit Johannes**
**Van Lennephof 53**
**NL-5343 JD OSS (NL)**
Inventor : **Egberink, Johannes Gerhardus**
**Joseph**
**Lisztgaarde 27**
**NL-5344 EB OSS (NL)**

**(74)** Representative : **Hermans, Franciscus G.M. et al**
**Patent Department AKZO N.V. Pharma Division P.O. Box 20**
**NL-5340 BH Oss (NL)**

## Description

The present invention relates to a new pharmaceutical preparation for local application comprising an aqueous solution of 11β-hydroxy-16α,17α,21-trimethylΔ$^{1,4}$-pregnadiene-3,20-dione (hereinafter called Org 6216 or the Steroid) and a β-cyclodextrine derivative.

More particularly the said new composition is particularly useful for ophthalmological purposes, viz. for the treatment of infections in or at the eyes.

In general, 11β-hydroxy steroids (corticosteroids) including the Steroid are known for their potent local antiinflammatory activity, see for example US patent 3,947,478. Moreover, Org 6216 did not expose any significant systemic effect when administered locally.

However, it is also generally known that most corticosteroids when applied to the eyes are held responsible for an unacceptable increase of intra ocular pressure (I.O.P.) which render them absolutely unsuitable for any ophthalmological application. Fortunately, however, it was recently found that contrary to other corticosteroids Org 6216 did not increase I.O.P., so that also an ophthalmological application of Org 6216 could be considered.

Org 6216, however, has one significant drawback which hitherto blocked virtually any practical application namely its extremely poor solubility in aqueous systems.

For local application in the form of injection preparations, eye-drops, ear-drops, nose-sprays or the like the desired concentration of in water could not be obtained. For topical application in the form of a gel, foams, cream or ointment (on water basis) large amounts of the Steroid were necessary in order to have very small amounts penetrated through the skin.

In general it is known that α,β and τ-cyclodextrins may form inclusion complexes with various organic or inorganic compounds and as a result may increase the solubility of these compounds. See e.g. the review article of Saenger in Angewandte Chemie Int. Ed. 19, 344 (1980).

It is Applicant's own experience and supported by literature data that the solubility of a compound in water may increase very roughly by a factor 2-500 provided said compound is able to form an inclusion-complex with one of the cyclodextrins.

Org 6216, however, would not benefit from such increase because its original solubility in water of approximately $5 \times 10^{-4}$ mg/ml would most ideally increase at most up to a level of about 0.1 mg/ml which is still insufficient for local administration of Org 6216 in general and more in particular for ophthalmological application. Practical solubility tests carried out with α,β and τ-cyclodextrin only confirmed this expectation.

Surprisingly, however, it was found that an inclusion complex formed between Org 6216 and 2,6dimethyl-β-cyclodextrin increased the solubility of the Steroid in water from the original level of $5 \times 10^{-4}$ mg/ml up to a level of about 10 mg/ml, which means an increase of the solubility with the unexpectedly high factor of 10,000.

This increase of Org 6216 solubility in water renders a practical application of the Steroid possible in pharmaceutical preparations for local administration and especially in ophthalmological preparations.

The present invention therefore encompasses a pharmaceutical preparation for local administration, and especially an ophthalmological preparation comprising an aqueous solution of 11β-hydroxy-16α,17α,21-trimethyl-Δ$^{1,4}$- pregnadiene-3,20-dione (the Steroid, Org 6216) and a partially alkylated β-cyclodextrin.

A partially alkylated β-cyclodextrin is a derivative of β-cyclodextrin (or cyclohepta amylose) in which one or more hydroxy groups are alkylated. Examples of such partially alkylated β-cyclodextrins are the 2 hydroxypropyl, 6-methyl, 2,3-dimethyl and in particular the 2,6-dimethyl-β-cyclodextrin.

The solubility of Org 6216 in water increases with increasing concentrations of partially alkylated β-cyclodextrin until this solubility reaches its level-off phase or maximal level. This maximal level is obtained using relatively large amounts of partially alkylated β-cyclodextrins, roughly 0.2-0.3 mmol. partially alkylated β-cyclodextrin per mililiter water.

The maximal solubility of Org 6216 in water using 2,6-dimethyl-β-cyclodextrin as the solubilizing agent amounts to about 9-10 mg/ml; at this level the 2,6-dimethyl-β-cyclodextrin concentration required is about 0,28 millimol per ml. A further increase of the cyclodextrin concentration contributes less effectively to a further improvement of the Steroid's solubility.

The graph shows the increase of the solubility of Org 6216 with increasing 2,6-dimethyl-β-cyclodextrin concentrations.

The solubility studies were performed according to the method described below which is well known in the literature. Excess amounts of Org 6216 were added to aqueous solutions of 2,6-dimethyl-β-cyclodextrin. These suspensions were shaken at room temperature for 5 days followed by an equilibration period of 3 days at room temperature. Then an aliquot was centrifuged and the supernatant was filtered through a 0.45 μm membrane filter (Millipore: HV4, Millex™). The samples were analysed with the Perkin Elmer Lambda 5 UV/VIS spectrophotometer. The absorbance was read and compared with a blanc and standard calibration curve. From these data the concentration of Org 6216 dissolved in water was calculated. After calculation of the amount of

EP 0 326 196 B1

steroid dissolved, a phase-solubility diagram was constructed by plotting the dissolved amounts of Org 6216 as a function of the concentration of 2,6-dimethyl-β-cyclodextrin (see Graph).

From the curve obtained the complex stability constant K was calculated using the formula:

$$K = \frac{slope}{S_o (1-slope)}$$

$S_o$ = saturation concentration of Steroid without 2,6-dimethyl-β-cyclodextrin.

slope = the slope of the initial part of the curve.

This complex-stability constant K is also a measure for the solubility of Org 6216 in water. The higher this complex-stability constant, the better is the stability of the complex and the higher is the solubility in water.

For comparative purposes the unexpected increase of solubility of Org 6216 in water using 2,6-dimethyl-β-cyclodextrin, is compared with other more common cyclodextrins. The results are described in the following table:

| Cyclodextrin reference compounds | Steroid Org 6216 |
|---|---|
| α-cyclodextrin<br>K (ml.mmol.$^{-1}$)<br>Solubility (mg/ml) | 380<br>$4 \times 10^{-2}$ |
| β-cylodextrin<br>K (ml.mmol.$^{-1}$)<br>Solubility (mg/ml) | 3400<br>$5 \times 10^{-2}$ |
| γ-cyclodextrin<br>K (ml.mmol.$^{-1}$)<br>Solubility (mg/ml) | 9600<br>$7 \times 10^{-2}$ |
| Part. alkylated cyclodextrin<br>2,6-dimethyl-β-cyclodextrin<br>K (ml.mmol$^{-1}$)<br>Solubility (mg/ml) | 58000<br>9 |
| Solubility without cyclodextrin, mg/ml | $7 \times 10^{-4}$ |

Besides the essential ingredients Org 6216, partially methylated β-cyclodextrin and water, the pharmaceutical preparation according to the invention may contain other active substances and/or pharmaceutical auxiliaries.

The preparation may optionally contain for example, preservatives, viscosity increasing agents, agents for rendering the solution isotonic, buffers for adapting the desired pH, surfactants etc., as well as the usual pharmaceutically acceptable carriers and/or diluents.

As already said the preparation according to the invention may be used for all kinds of local administration, e.g. as injection preparation, intranasal preparation, ear-drops, topical preparation (gel, ointment, foam, cream), but is preferably used for ophthalmological purposes.

A preparation for local administration is a preparation that is locally active and does not exert a significant systemic effect.

The concentration of Org 6216 in the pharmaceutical preparation according to the invention may vary somewhat depending upon the way of administration. The usual concentration, however, varies between 1 and 10 mg and more particularly between 5 and 10 mg per mililiter water. For ophthalmological purposes where usually only one or two drops are applied the concentration of ORG 6216 is preferably as high as possible, viz. about 9-10 mg per ml water.

3

## Examples

1. Topical gel

| | |
|---|---|
| 2,6-dimethyl-$\beta$-cyclodextrin | 50.0 g |
| Org 6216 | 0.9 g |
| pectin | 5.0 g |
| sugar | 30.0 g |
| water to | 100 ml |

prepare complex, add pectin and dissolve, add sugar adjust pH to 3-4.

2. o/w emulsion (w = water phase, o = oil phase)

| | | |
|---|---|---|
| 2,6-dimethyl-$\beta$-cylodextrin | 185 g | w |
| Org 6216 | 3.3 g | w |
| sodium laurylsulphate | 10 g | w |
| stearyl alcohol | 250 g | o |
| white petrolatum | 250 g | o |
| methylhydroxybenzoate | 0.7 g | w |
| propylhydroxybenzoate | 3.7 g | o |
| water | 370 g | |
| propylene glycol | 120 g | w |
| | -------- | |
| | 1192.7 g | |

prepare oil phase and water phase separately at elevated temperatures then, add oil phase to water phase and homogenize.

3. Opthalmic solution (eye drop)

| | |
|---|---|
| Org 6216 | 4.5 g |
| 2,6-dimethyl-$\beta$-cyclodextrin | 250.0 g |
| polyvinylalcohol | 7.0 g |
| $Na_2HPO_4$ | 3.8 g |
| $NaH_2PO_4$ | 0.8 g |
| Chlorobutanol | 2.5 g |
| NaCl to | isotonic |
| Water for injection to | 500 ml |

prepare complex, add other components, adjust to volume.

4. <u>Injectable preparation</u>

| | |
|---|---|
| Org 6216 | 9 mg |
| 2,6-dimethyl-$\beta$-cyclodextrin | 500 mg |
| NaOH/HCl to | pH 7 |
| Mannitol to | isotonic |
| Water for injection to | 1.0 ml |

prepare complex, add other components, adjust to volume.

5. <u>Otic preparation (ear)</u>

| | |
|---|---|
| 2,6-dimethyl-$\beta$-cyclodextrin | 125 g |
| Org 6216 | 2.25 g |
| benzylalcohol | 10 g |
| sodium carboxymethylcellulose | 0.025 g |
| propylene glycol | 25.0 g |
| NaCl to | isotonic |
| NaOH/HCl to | pH 7 |
| Water for injection to | 250 ml |

6. <u>Topical foam</u>

<u>Emulsion base</u>

| | |
|---|---|
| Org 6216 | 7.1 g |
| 2,6-dimethyl-$\beta$-cyclodextrin | 394.2 g |
| miristic acid | 12.6 g |
| stearic acid | 42.0 g |
| cetylalcohol | 3.9 g |
| lanolin | 1.6 g |
| isopropylmyristate | 12.6 g |
| triethanolamine | 26.3 g |
| glycerin | 37.1 g |
| polyvinylpyrrolidone | 2.7 g |
| water | 700.0 ml |

<u>Final</u>

960 g emulsion base 40 g hydrocarbon propellant A-46

7. <u>Nasal preparation</u>

| | | |
|---|---|---|
| Org 6216 | 90 | mg |
| 2,6-dimethyl-$\beta$-cyclodextrin | 5000 | mg |
| benzalkoniumchloride | 0.001 | mg |
| EDTA | 0.01 | mg |
| NaOH/HCl to | pH 7 | |
| Mannitol to | iso-osmotic | |
| Water for injection to | 10 | ml |

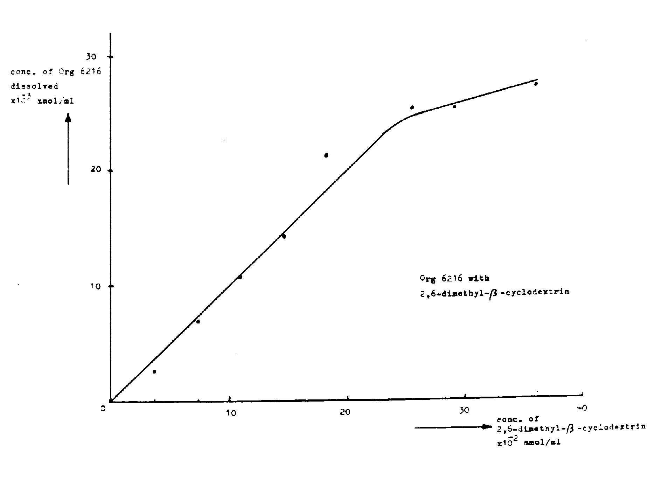

## Claims

### Claims for the following Contracting States : AT, BE, CH, DE, FR, GB, IT, LI, LU, NL, SE

1. A pharmaceutical preparation for local administration comprising an aqueous solution of 11-$\beta$-hydroxy-16$\alpha$,17$\alpha$, 21-trimethyl-$\delta^{1,4}$-pregnadiene 3,20-dione and a partially alkylated $\beta$-cyclodextrin.

2. Preparation according to claim 1 comprising 2,6-dimethyl-$\beta$-cyclodextrin as the partially alkylated $\beta$-cyclodextrin.

3. Preparation according to claim 1 or 2 intended for opthalmological purposes.

4. Preparation according to claim 3 comprising 5-10 mg of 11-$\beta$-hydroxy-16$\alpha$,17$\alpha$, 21-trimethyl-$\delta^{1,4}$-pregnadiene 3,20-dione per ml water.

### Claims for the following Contracting Sates : GR, SP

1. A process of manufacturing a pharmaceutical preparation for local administration comprising dissolving 11-$\beta$-hydroxy-16$\alpha$,17$\alpha$, 21-trimethyl-$\delta^{1,4}$-pregnadiene 3,20-dione into an aqueous solution containing a partially alkylated $\beta$-cyclodextrin.

2. The process according to claim 1 wherein 2,6-dimethyl-$\beta$-cyclodextrin is the partially alkylated $\beta$-cyclodextrin.

3. The process of claim 1 or claim 2 wherein said pharmaceutical preparation is intended for opthalmological purposes.

4. The process according to claim 3 wherein the resulting pharmaceutical preparation comprises 5-10 mg of 11-$\beta$-hydroxy-16$\alpha$,17$\alpha$, 21-trimethyl-$\delta^{1,4}$-pregnadiene 3,20-dione per ml water.

## Patentansprüche

### Patentansprüche für folgende Vertragsstaaten : AT, BE, CH, DE, FR, GB, IT, LI, LU, NL, SE

1. Pharmauzeutisches Präparat zur örtlichen Verabreichung, enthaltend eine wässerige Lösung von 11 $\beta$-Hydroxy-16$\alpha$,17$\alpha$,21-trimethyl- $\delta^{1,4}$-pregnadien-3,20-dion und ein partiell alkyliertes $\beta$-Cyclodextrin.

2. Präparat nach Anspruch 1, enthaltend 2,6-Dimethyl-$\beta$-Cyclodextrin als das partiell alkylierte $\beta$-Cyclodextrin.

3. Präparat nach Anspruch 1 oder 2, bestimmt für ophthalmologische Zwecke.

4. Präparat nach Anspruch 3, enthaltend 5 bis 10 mg 11$\beta$-Hydroxy-16 $\alpha$ ,17$\alpha$, 21-trimethyl- $\delta^{1,4}$-pregnadien-3,20-dion pro Milliliter Wasser.

### Patentansprüche für folgende Vertragsstaaten : GR, SP

1. Verfahren zur Herstellung eines pharmazeutischen Präparates zur örtlichen Verabreichung, dadurch gekennzeichnet, dass 11$\beta$-Hydroxy-16$\alpha$,17$\alpha$,21-trimethyl-$\delta^{1,4}$-pregnadien-3,20-dion in einer wässerigen Lösung, welche ein partiell alkyliertes $\beta$-Cyclodextrin enthält, aufgelöst wird.

2. Verfahren nach Anspruch 1, dadurch gekennzeichnet, dass 2,6-Dimethyl-$\beta$-cyclodextrin das partiell

alkylierte β-Cyclodextrin ist.

3. Verfahren nach Anspruch 1 oder 2, dadurch gekennzeichnet, dass das pharmazeutische Präparat für ophthalmologische Zwecke bestimmt ist.

4. Verfahren nach Anspruch 3, dadurch gekennzeichnet, dass das resultierende pharmazeutische Präparat 5 bis 10 mg 11β-Hydroxy-16α,17α, 21-trimethyl-$\delta^{1,4}$-pregnadien-3,20-dion pro Milliliter Wasser enthält.

**Revendications**

**Revendications pour les Etats contractants suivants : AT, BE, CH, DE, FR, GB, IT, LI, LU, NL, SE**

1. Préparation pharmaceutique pour administration locale, comprenant une solution aqueuse de 11β-hydroxy-16α,17α,21-triméthyl-$\Delta^{1,4}$-prégnadiène-3,20-dione et d'une β-cyclodextrine partiellement alkylée.

2. Préparation selon la revendication 1, comprenant de la 2,6-diméthyl-β-cyclodextrine en tant que β-cyclodextrine partiellement alkylée.

3. Préparation selon la revendication 1 ou 2, destinée à des usages ophtalmologiques.

4. Préparation selon la revendication 3, comprenant 5 à 10 mg de 11β-hydroxy-16α,17α,21-triméthyl-$\delta^{1,4}$-prégnadiène-3,20-dione par ml d'eau.

**Revendications pour les Etats contractants suivants : GR, SP**

1. Procédé de fabrication d'une préparation pharmaceutique pour administration locale, consistant à dissoudre de la 11β-hydroxy-16α,17α,21-triméthyl-$\delta^{1,4}$-prégnadiène-3,20-dione dans une solution aqueuse contenant une β-cyclodextrine partiellement alkylée.

2. Procédé selon la revendication 1, dans lequel la 2,6-diméthyl-β-cyclodextrine est la β-cyclodextrine partiellement alkylée.

3. Procédé selon la revendication 1 ou 2, dans lequel ladite préparation pharmaceutique est destinée à des usages ophtalmologiques.

4. Procédé selon la revendication 3, dans lequel la préparation pharmaceutique comprend 5 à 10 mg de 11β-hydroxy-16α,17α,21-triméthyl-$\Delta^{1,4}$-prégnadiène-3,20-dione par ml d'eau.